(19) Europäisches Patentamt
European Patent Office
Office européen des brevets

(11) **EP 4 174 535 A1**

(12) **EUROPEAN PATENT APPLICATION**

(43) Date of publication:
**03.05.2023 Bulletin 2023/18**

(21) Application number: **21204739.3**

(22) Date of filing: **26.10.2021**

(51) International Patent Classification (IPC):
**G02B 3/10** (2006.01)    **G02C 7/06** (2006.01)

(52) Cooperative Patent Classification (CPC):
**G02B 3/10; A61F 2/16;** G02C 7/042; G02C 2202/20

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Universiteit Antwerpen
2000 Antwerpen (BE)**
• **Universitair Ziekenhuis Antwerpen
2650 Edegem (BE)**

(72) Inventors:
• **VAN DYCK, Dirk
2630 Aartselaar (BE)**
• **TASSIGNON, Marie-José
2600 Berchem (BE)**
• **GOBIN, Laure
92410 Ville d'Avray (FR)**

(74) Representative: **Beck, Michaël Andries T.
IPLodge
Technologielaan 9
3001 Heverlee (BE)**

(54) **INTRAOCULAR LENS**

(57)    The application pertains to an intraocular lens (100) for contributing to the focusing of incident light onto a retina (70) of a user, the intraocular lens (100) comprising: a first surface (110), and a second surface (120), wherein said first surface (110) is an aspheric surface configured to refractively focus said incident light, said first surface (110) exhibiting a non-uniform refractive power; wherein said second surface (120) is provided with a diffraction generating profile in the form of zero order Bessel function configured to spatially separate said incident light into at least a first portion having a first spatial frequency characteristic and a second portion having a second spatial frequency characteristic; and wherein said spatial separation and said non-uniform refractive power of said first surface (110) cause said first portion to be refracted by said first surface (110) at a first level of refractive power and said second portion to be refracted by said first surface (110) at a second level of refractive power, said first level of refractive power and said second level of refractive power being different.

Figure 2

**Description**

**Field of the Invention**

The present invention relates to the field of intraocular lenses, in particular intraocular lenses with extended depth-of-focus.

**Background**

The refracting power of the unaltered human eye is defined by the cornea, the crystalline lens, and the axial length of the eyeball, which cooperate to produce an image on the retina. Unmatched parameters will lead to myopia (nearsightedness) and hyperopia (farsightedness), which can be treated by providing the appropriate dioptric correction in the form of eyeglasses or contact lenses. In humans, the lens is capable of adjustment (accommodation), by the action of the ciliary muscle, allowing the individual to focus on objects in a wide range of distances.

An individual's ability to accommodate is impaired with age in various different ways, leading to difficulties in reading, even when properly corrected for far distance. The loss of accommodation is due to ageing of the crystalline lens, which first becomes hard and then opaque (this is cataract). Visual axis transparency can be restored by replacing the natural crystalline lens by an implanted intraocular lens.

The onset of age-related loss of accommodation, known as presbyopia, leads to a need for different powers of dioptric correction, to achieve or restore sharp vision at near, intermediate, and far distances. This has led to the development of bifocal and multifocal (progressive) eyeglasses and contact lenses. In the field of intraocular lenses, where accommodation is rendered even more difficult due to the challenges of transmitting forces from the ciliary muscle to the artificial intraocular lens, a similar approach has been attempted. This has led to two major different approaches: the accommodative intraocular lenses, where lens elements might move by transmission of ciliary forces, and the pseudo-accommodative intraocular lenses, including the bifocal, trifocal, and (continuous) extended depth-of-focus intraocular lenses. So far, accommodative intraocular lenses are not clinically successful but the pseudo-accommodative intraocular lenses have shown quite some useful progress during the last twenty years.

It is a known disadvantage of multifocal intraocular lenses aiming at covering a 3 diopter range of focus for near vision that they cause halos and other undesirable visual artefacts, while extended depth-of-focus intraocular lenses aiming at covering a 1.5 diopter range of focus for near vision cause less discomfort but tend to perform less well in the near vision. Both types may also reduce contrast sensitivity.

Accordingly, there is a need for further improvements in the field of pseudo-accommodative intraocular lenses that use optical techniques to compensate for the lack of accommodation.

**Summary**

Embodiments of the present invention are based *inter alia* on the insight of the inventors that a better depth-of-focus experience can be obtained in an intraocular lens by combining refractive and diffractive optics using both sides of the lens.

According to an aspect of the present invention, there is provided an intraocular lens for contributing to the focusing of incident light onto a retina of a user, the intraocular lens comprising:

-   a first surface, and
-   a second surface,

   wherein said first surface is configured to refractively focus said incident light, said first surface exhibiting a non-uniform refractive power;
   wherein said second surface is provided with a diffraction generating profile configured to spatially separate said incident light into at least a first portion having a first spatial frequency characteristic and a second portion having a second spatial frequency characteristic;
   and wherein said spatial separation and said non-uniform refractive power of said first surface cause said first portion to be refracted by said first surface at a first level of refractive power and said second portion to be refracted by said first surface at a second level of refractive power, said first level of refractive power and said second level of refractive power being different.

In use, one of said first surface and said second surface faces the retina, and the other one of said first surface and said second surface faces away from said retina (i.e. it faces the cornea).

It is known to use at least the first surface as a refractive surface to contribute to the focusing of incident light onto the retina of the user. The inventors have found that it is advantageous to use the second surface as a diffractive separator that splits the entrance wave into different components that are refracted at different levels of optical power, thus leading to an extension of the perceived depth of focus.

In an embodiment, the intraocular lens according to the present invention is shaped as a biconvex lens.

It is an advantage of this embodiment that the desired level of refraction can be obtained with a relatively thin lens, which facilitates the insertion of the lens into the eye.

In an alternative embodiment, the intraocular lens according to the present invention is shaped as a planoconvex lens, wherein the first surface is convex.

While it is common in the prior art to use both surfaces of the intraocular lens for refraction, the inventors have found that it is advantageous to use the second surface not as an extra lens but only as a diffractive separator.

While this specific embodiment is easy to model and has been used in some of the simulations presented in the remainder of this description, it does present a greater thickness than a biconvex lens for the same dioptric power, which needs to be taken into account when selecting the most suitable design for any particular application.

In an embodiment of the intraocular lens according to the present invention, the non-uniform refractive power of said first surface presents a characteristic of positive spherical aberration.

It is an advantage of this embodiment that spherical aberration, which would otherwise be considered to an undesirable flaw of spherical lenses, can be advantageously exploited to deliberately direct a portion of the incident light to an area of the lens where the optical power is stronger, in order to increase the perceived depth of focus.

In an embodiment of the intraocular lens according to the present invention, said diffraction generating profile exhibits a circular symmetry. In a particular embodiment, said diffraction generating profile is shaped according to a Bessel function of the first kind.

The "Bessel lens" is in fact a tandem lens in which the second surface acts as a beam selector that extracts a cone of light, which, by virtue of the optical non-uniformity (in particular, the spherical aberration) of the first surface, is then focused to a different but complementary focal range. In other words, a portion of the incident light passes through the central portion of the first surface in the regular manner while an angular passband (cone) of waves is diffracted at an higher angle.

It is an advantage of this particular way of beam selection that all the extracted light is exactly concentrated on the desired focus without any unwanted loss of intensity so that it can optimally combine focal depth with image quality.

In a particular embodiment, the diffraction generating profile is shaped according to a zero-order Bessel function of the first kind, $z(r) = A \cdot J_0(r/P)$, wherein a radial scaling factor $P$ is in the range between 90 $\mu$m and 900 $\mu$m and a profile depth scaling factor $A$ is in the range between 100 nm and 1000 nm.

The inventors have found that a profile shaped according to those parameters yields an advantageous level of spatial separation.

In an embodiment of the intraocular lens according to the present invention, the intraocular lens comprises a haptic part that surrounds the optical part of the lens and further contains a groove of such shape to accommodate the anterior and posterior capsules of the lens bag.

It is particularly advantageous to apply the present invention in combination with the bag-in-the-lens technique. The bag-in-the-lens technique is unmatched in its ability to align the intraocular lens with the optical axis of the user's eye, which avoids the formation of visual artefacts that might otherwise result from a misalignment between the cornea, and the respective surfaces of the intraocular lens.

**Brief Description of the Figures**

These and other features and advantages of embodiments of the present invention will now be described in more detail with reference to the accompanying drawings, in which:

- Figure 1 schematically represents a sagittal cross-section of a human eye, provided with an intraocular lens according to an embodiment of the present invention;
- Figure 2 is a plot of a diffraction generating profile according to a Bessel function, as may be provided on the anterior surface of an intraocular lens according to an embodiment of the present invention;
- Figure 3 is a simulated diagram of the diffraction pattern (point spread function) generated by the surface profile of Figure 2;
- Figure 4 illustrates the placement of an intraocular lens by means of the bag-in-the-lens technique;
- Figure 5 schematically illustrates the set-up of a simulation designed to show the amount of defocus experienced by the user of a traditional biconvex intraocular lens;
- Figure 6 is a spot diagram resulting from the set-up of Figure 5;
- Figure 7 plots the relationship between object distance and defocus in the set-up of Figure 5;

- Figure 8 schematically illustrates the set-up of another simulation designed to test and validate the effect obtained by the intraocular lens according to the present invention;
- Figure 9 is a spot diagram resulting from the set-up of Figure 8;
- Figures 10 and 11 plot the spot size versus image distance for different aspherical shapes for a simulation model with and without a diffraction generating profile on the posterior surface, respectively;
- Figure 12 presents a direct comparison of the spot sizes for a simulation model with and without a diffraction generating profile on the posterior surface;
- Figures 13 and 14 plot the point spread functions at different image distances for a simulation model with and without a diffraction generating profile on the posterior surface, respectively; and
- Figures 15 and 16plot the modulation transfer functions at different image distances for a simulation model with and without a diffraction generating profile on the posterior surface, respectively.

## Description of Embodiments

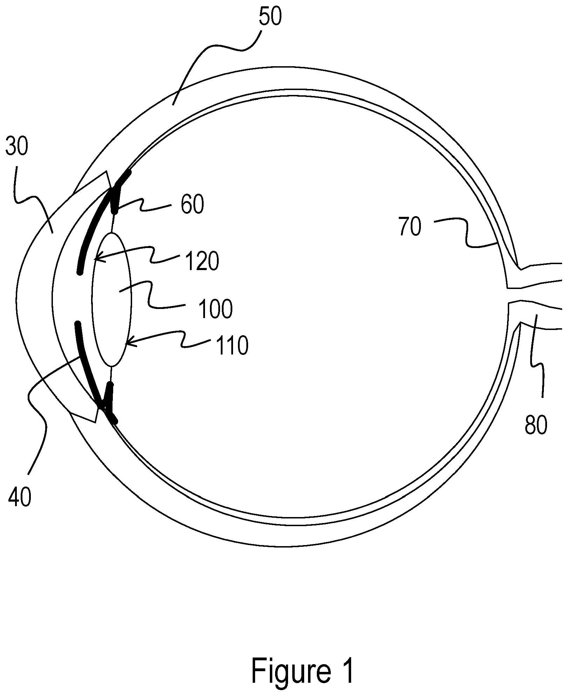

In its most general form, the present invention provides an intraocular lens for contributing to the focusing of incident light onto a retina of a user. To understand the optical role and function of the intraocular lens according to the present invention, a sagittal cross-section of a human eye, provided with an intraocular lens according to an embodiment of the present invention is schematically represented in Figure 1. The reader will understand that only the major elements of the eye are represented as reference points for the purpose of elucidating the invention and that many anatomical details have accordingly been omitted or simplified.

Assuming the illustrated orientation of the eye, light travels from left to right, entering the eye through the cornea **30,** which provides a first amount of refractive power (typically around 43 diopters). The cornea **30** is surrounded by the sclera **50.** The size of the pupil (and hence, the amount of light allowed to reach the lens) is modulated by the iris **40.** Light travels from the cornea **30,** through the pupil, to the intraocular lens **100,** whereby the intervening space is filled with vitreous humor (not illustrated). The intraocular lens **100** takes up the role of the crystalline lens in the unaltered eye and provides a second amount of refractive power (for the crystalline lens in a healthy unaltered eye, this is typically around 18 diopters).

The light that passes through the intraocular lens **100** is projected onto the retina **70.** The optical nerve **80** passes the detected images on to the brains.

The intraocular lens **100** is positioned inside the eye, where it is attached to the lens capsule, which is in turn suspended from the ciliary muscle **60.** The intraocular lens (or at least its optical part) may be made of any transparent biocompatible material, including materials currently used for rigid optics, such as polymethylmethacrylate, or deformable optics, such as silicone polymeric materials, acrylic polymeric materials, hydrogel forming polymeric materials and mixtures of these materials or the like.

The intraocular lens **100** has two main surfaces or sides, which have been referred to hereinbefore as the "first surface" and the "second surface". In use, one of said first surface and said second surface faces the retina, and the other one of said first surface and said second surface faces away from said retina (i.e. it faces the cornea).

Without loss of generality, the figures and the remainder of this description will assume the orientation of the implanted intraocular device to be as follows: the intraocular device is implanted in such a way that the first surface **110** is the posterior surface, which faces the retina **70,** and the second surface **120** is the anterior surface, which faces the cornea **30.**

While we describe embodiments of the invention hereinbelow in a context in which, in use, the first surface **110** faces the retina **70** and the second surface **120** faces away from the retina **70** (i.e., it faces the cornea **30**), the reader will understand that this way of describing the embodiments is not meant to limit the scope or applicability of the invention. The use of the notation "first (posterior) surface" and "second (anterior) surface" in the present description is only intended to assist the reader in identifying the first surface and the second surface in the figures. Accordingly, it shall be understood that the intraocular lens **100** may also be implanted in the inverse orientation (not illustrated), with the same technical effects.

According to the invention, the first (posterior) surface **110** is configured to refractively focus the incident light and exhibits a non-uniform refractive power. Further according to the invention, the second (anterior) surface **120** is provided with a diffraction generating profile configured to spatially separate the incident light into at least a first portion having a first spatial frequency characteristic and a second portion having a second spatial frequency characteristic.

The reader will note that the respective curvatures of the second (anterior) surface **120** and the first (posterior) surface **110** are not drawn to scale in Figure 1. In view of their different roles, these surfaces may exhibit different curvatures. Preferably, the intraocular lens **100** is a biconvex lens. Alternatively, the intraocular lens **100** may be a planoconvex lens, whereby the first (posterior) surface **110** is convex.

The spatial separation occurring at the second (anterior) surface **120** and the non-uniform refractive power of the first (posterior) surface **110** are designed to cooperate in such a way that the first portion of the incident light is refracted by the first (posterior) surface **110** at a first level of refractive power and the second portion of the incident light is refracted by the first (posterior) surface **110** at a second level of refractive power. The first level of refractive power and the second level of refractive power are different.

Thus, the second (anterior) surface **120** is used as a diffractive separator that splits the entrance wave into different components that are refracted at different levels of optical power, leading to an extension of the perceived depth of focus. The diffractive separation is obtained by a diffraction generating profile (i.e., a grating defined by height or thickness variations of the surface) provided on the second (anterior) surface **120.** The thickness variations cause variations in phase that build the diffraction pattern. The surface profile is preferably generated during the manufacturing of the intraocular lens **100,** for example by means of molding or lathing.

Terms such as "first portion", "second portion", and "separator" are not to be construed as limiting the invention to embodiments where incoming light is divided into disjoint bundles in order to be refracted by separate portions of the first (posterior) surface having discretely varying optical power. Both the spatial separation and the variation in optical power of the first (posterior) surface may be of a continuous nature.

In an embodiment of the intraocular lens **100** according to the present invention, the non-uniform refractive power of the first (posterior) surface **110** presents a characteristic of positive spherical aberration. Such a surface may be defined by expressing the displacement $z(r)$ of the surface from the vertex at a distance $r$ from the axis in function of a radius of curvature $R$, a conic constant $k$, and series of aspherical coefficients $a_i$, as follows:

$$z(r) = \frac{r^2}{R\left(1 + \sqrt{1 - (1+k)\frac{r^2}{R^2}}\right)} + a_4 r^4 + a_6 r^6 + \cdots$$

The inventors have experimentally observed that a slight increase in spherical aberration causes a slight extension of the depth of focus in known intraocular lenses. The reason is that the spherical aberration makes the lens stronger for the higher spatial frequencies. But because this affects only a small fraction of the light, the focal extension is rather small. Generally, increasing the spherical aberration does not tend to improve this, because it also affects the main spatial frequencies and deteriorates the image quality.

In the context of the present invention, however, the diffractive beam separator synergistically cooperates with the spherical aberration to obtain a surprisingly pronounced focal extension effect. Because the fraction of high-frequency components can be arbitrarily large, the total focal range can be extended significantly.

The diffraction generating profile preferably exhibits a circular symmetry. More preferably, the diffraction generating profile is shaped according to a Bessel function of the first kind. According to the Fraunhofer theory of optical diffraction, the diffracted wave is the Fourier transform of the profile of the scattering surface. If the desired wave form is a ring shape, the profile of the scattering surface must be the inverse Fourier of a ring shape, in which case the radial profile is a $J_0(r)$ Bessel function.

In order to be effective in the context of the present invention, the Bessel profile must be judiciously dimensioned. Given the rotational symmetry, there are only two parameters that determine the "scale" of the Bessel profile: a factor $A$ that scales the pattern in the z-direction (representing the amplitude of the pattern, perpendicular to the surface, i.e. the "depth" of the grooves of the profile) and a factor $P$ that scales the pattern in the radial direction (representing the period of the pattern, along the surface, i.e. the "width" of the grooves of the profile). The profile can then be expressed as:

$$z(r) = A \cdot J_0(r/P)$$

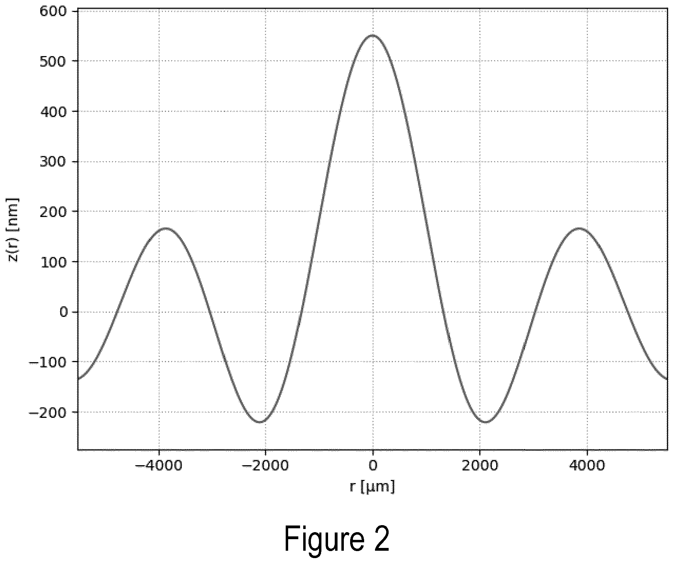

Figure 2 is a plot of a diffraction generating profile according to a Bessel function as defined above, with $A$ = **550nm** and $P$ = **550μm.** The plot runs from $r$ = **-5500μm** to $r$ = **5500μm,** which corresponds to the extent of an intraocular lens with an exemplary diameter of 11 mm. Note that different scales were used for the $r$-direction and for the $z$-direction for clarity purposes, thus exaggerating the depth of the grooves.

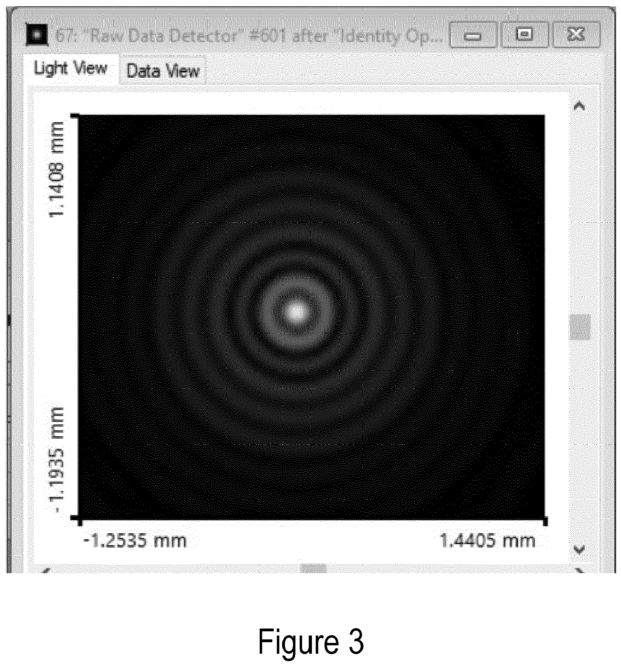

Figure 3 is a simulated diagram of the diffraction pattern (point spread function) generated by the surface profile of Figure 2, as seen at 1 m distance. The deviation of portions of the incident light in concentric rings is clearly visible, which shows that the desired spatial separation is achieved by the surface profile (while the appearance of multiple diffraction rings cannot be avoided with the profile periodicity described above, as it generates rings well below the diffraction limit, the amplitude of the second and higher-order rings is sufficiently small by virtue of the very small depth of the surface oscillations, which makes the diffraction generating profile a weak phase object).

While the above profile, defined by $A$ = **550nm** and $P$ = **550μm,** is a preferred embodiment, $A$ and $P$ may be varied over a wide range of values in view of the desired magnitude of the spatial separation and in further view of manufacturability considerations. *A* is preferably in the range between 100 nm and 1000 nm, and more preferably in the range between 300 nm and 700 nm. Specific values of *A* that have been used in simulations by the inventors and that may be used in embodiments of the present invention include 100 nm, 250 nm, 550 nm, 700 nm, 750 nm, 818 nm, 850 nm, and 1000 nm. *P* is preferably in the range between 90 $\mu$m and 900 $\mu$m, though values above 100 nm are preferred. Specific values of *P* that have been used in simulations by the inventors and that may be used in embodiments of the present invention include 91 $\mu$m, 250 $\mu$m, 300 $\mu$m, 400 $\mu$m, 550 $\mu$m, 600 $\mu$m, and 850 $\mu$m.

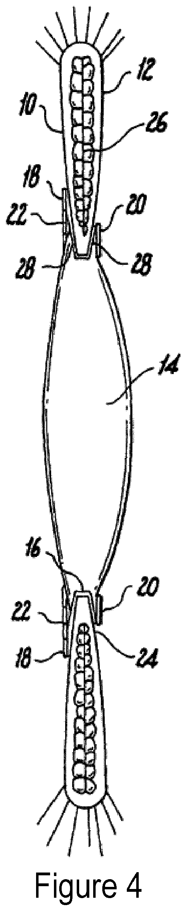

Figure 4 illustrates the placement of an intraocular lens in an eye by means of the bag-in-the-lens technique. European patent application no. EP 0 916 320 A1, by one of the present inventors, discloses an intraocular lens having a haptic part that surrounds the optical part of the lens and further contains a groove of such shape to accommodate the anterior and posterior capsules of the lens bag after anterior capsulorhexis, extracapsular cataract extraction and posterior capsulorhexis. The lens can be inserted in a calibrated, circular and continuous combined anterior and posterior capsulorhexis, slightly smaller than the inner circumference of the groove as to induce a stretching of the rims of the capsular openings, thus preventing secondary opacification of the capsules, allowing a very stable fixation of the intraocular lens and ensuring a tight separation between the anterior and posterior segment of the eye. This principle of insertion is called the bag-in-the-lens technique, in contrast with the classical lens-in-the-bag technique.

It is particularly advantageous to apply the present invention in combination with the bag-in-the-lens technique. Accordingly, as shown in Figure 4, an embodiment of the intraocular lens according to the present invention designed to be used with this technique further comprises a haptic part **15, 20** that surrounds the optical part **14** of the lens **100** and further contains a groove **16** of such shape to accommodate the anterior **10** and posterior **12** capsules of the lens bag. As further shown in Figure 4, optional perforations **22** of variable number and size may be added to the anterior **18** or posterior lip **20** of the haptic part to allow easier manipulation of the intraocular lens.

Because the diameters of both the anterior and posterior capsulorhexis are identical but slightly smaller than the smaller diameter of the lens optic **16,** the capsular openings will be stretched when inserting the lens, thus providing a tight junction **28** around the intraocular lens and a close space **26** or environment that contains the remaining lens epithelial cells of the lens bag. Contraction of the lens epithelial cells containing smooth muscle elements, will provide circular tractional folds **24** in the merged anterior and posterior capsules at the level of lens groove, further improving the tight junction **28** between the capsules and intraocular lens.

The efficacy of the invention will now be shown by referring to computer simulations of a number of reference set-ups and embodiments.

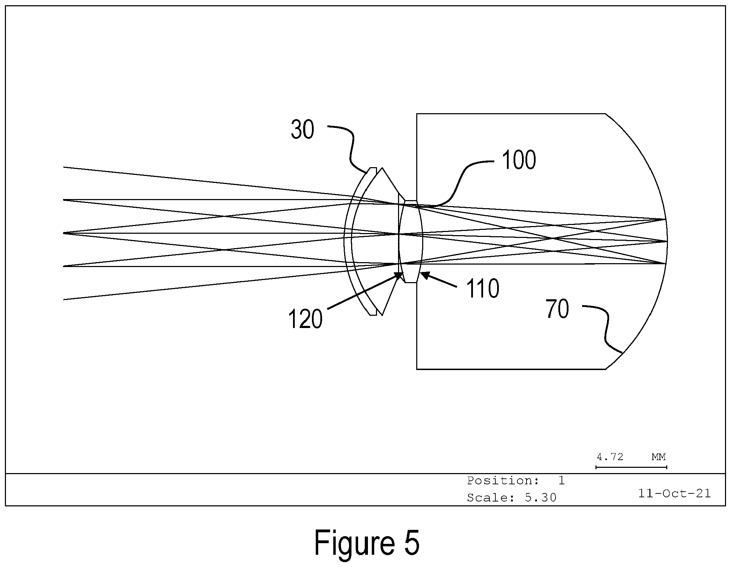

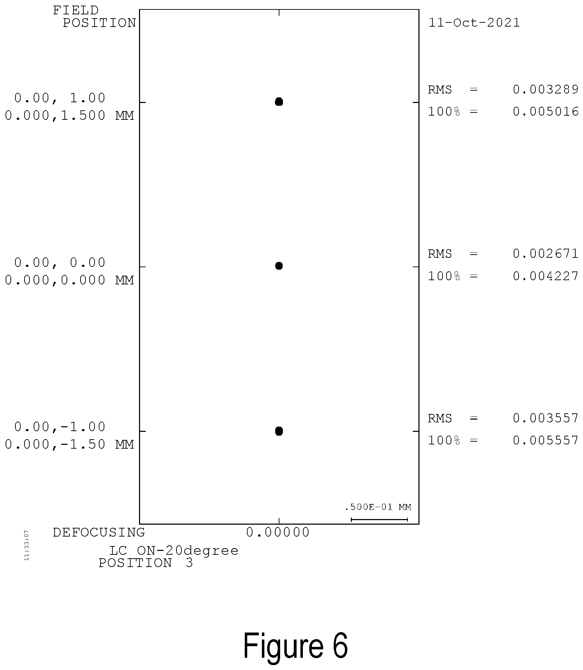

Figure 5 schematically illustrates the set-up of a simulation designed to show the amount of defocus experienced by the user of a traditional biconvex intraocular lens, when a nearby object is viewed. This set-up models the cornea **30** and a biconvex intraocular lens **100** having a first (posterior) surface **110** facing the retina **70** and a second (anterior) surface **120** facing the cornea **30**. The radius of curvature is $\pm$8.485 mm at the respective surfaces, yielding an optical power of +34.2 D, which is appropriate for distance vision. The first (posterior) surface **110** is aspherical with a nominal conic constant *k* = **-1.158** and an aspheric coefficient $a_4$ = **-0.000559,** while the second (anterior) surface **120** is aspherical with a nominal conic constant *k* = **-1.420** and an aspheric coefficient $a_4$ = **-0.000310.** Light beams from infinity enter through an entrance pupil having a diameter set at 3 mm. Figure 6 is a spot diagram resulting from the set-up of Figure 5.

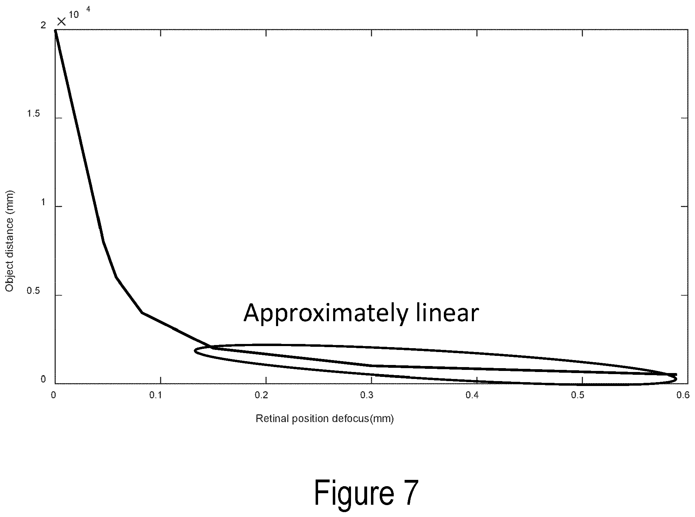

As this set-up is optimized for light arriving from an "infinite distance" (i.e. parallel rays, representing distance vision), a defocus will occur when the object being imaged is closer to the eye - this defocus can be expressed as a shift in retinal position or as a required amount of change in optical power. As shown in Figure 7, the retinal position defocus is approximately linear for object distances up to 20 m, ranging from 0.2 mm to 0.6 mm.

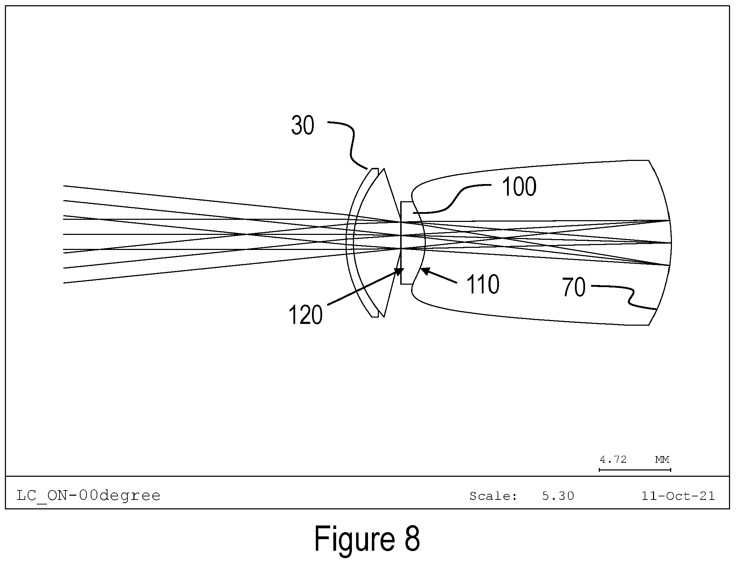

Figure 8 schematically illustrates the set-up of a simulation designed to test and validate the effect obtained by the intraocular lens **100** according to the present invention. To assess the effect of the invention, a first model, including only the refraction taking place at the cornea **30** and at the first (posterior) surface **110,** is compared with a second model that additionally includes the effect of the diffraction generating profile on the second (anterior) surface **120**. In both models, a planoconvex lens is provided with a radius of curvature of -3.938 mm at the first (posterior) surface **110,** yielding the same total optical power as the biconvex lens of the reference set-up of Figure 5 (+34.2 D). The first (posterior) surface **110** is aspherical with a nominal conic constant *k* = **-9.750** and aspheric coefficients $a_4$ = **-0.0110** (to be varied in the simulations) and $a_6$ = **-0.0009097.** Light beams enter through an entrance pupil having a diameter of 3 mm.

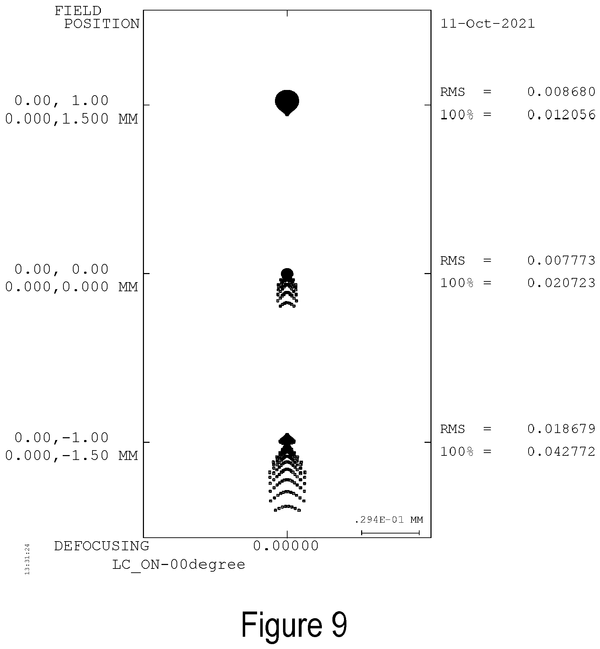

Figure 9 is a spot diagram resulting from the set-up of Figure 8. Comparing this diagram with the diagram of Figure 6, it can be seen that the planoconvex lens of the set-up of Figure 8, without the diffraction generating profile, has optical properties that are inferior to those of the biconvex lens of the set-up of Figure 5 (notably, larger spot size).

It is an object of embodiments of the present invention to provide increased depth of focus in order to overcome the aforementioned defocus at least in part. To that end, the second (anterior) surface **120** of the intraocular lens **100** according to the invention is provided with a surface profile designed to generate a diffraction pattern resulting in annular spatial separation of the incident light, as described above. For the purposes of the simulation, a profile according to the zero-order Bessel function of the first kind $J_0(r)$ was used in the second model, which was approximated by a polynomial with terms up to and including degree $r^6$.

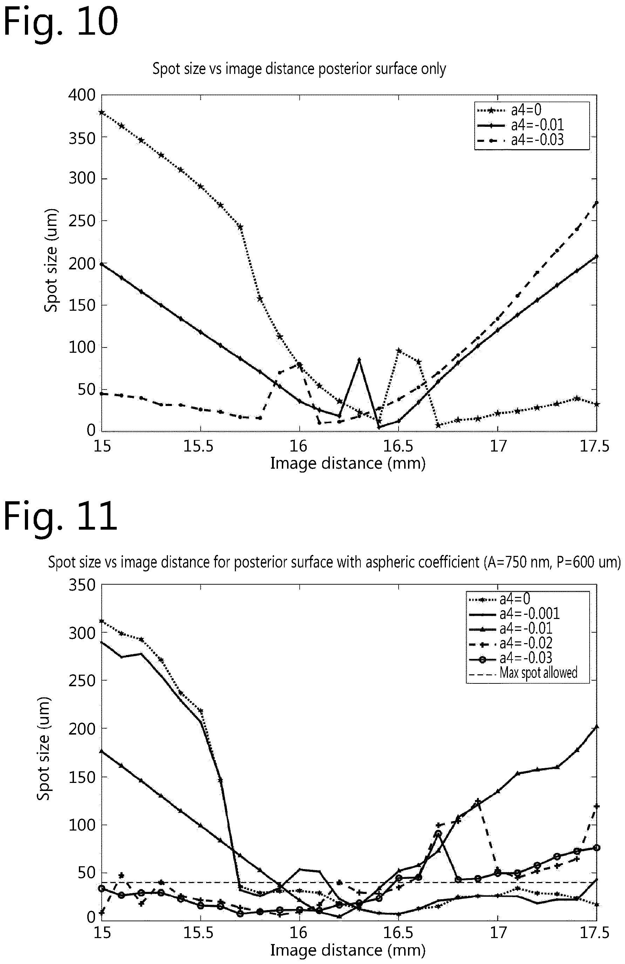

Figure 10 plots the spot size versus image distance in the first model, for three different values of $a_4$. This chart shows that the focus is optimal (spot size is minimal) in a narrow range of image distances between 16.0 mm and 16.5 mm for $a_4$ = **0.01,** and that the focal zone shifts towards shorter image distances with increasing values of $a_4$.

Figure 11 plots the spot size versus image distance in the second model, for five different values of $a_4$. The Bessel function used for the surface profile of the second (anterior) surface **120** was scaled as described above with $A$ = **750nm** and $P$ = **600$\mu$m.** This chart shows that acceptable focus (spot size below a predetermined threshold) can be obtained for a broader range of image distances for all selected values of $a_4$. The presence of the diffraction generating profile, in particular the Bessel surface profile, thus clearly improves the depth of focus.

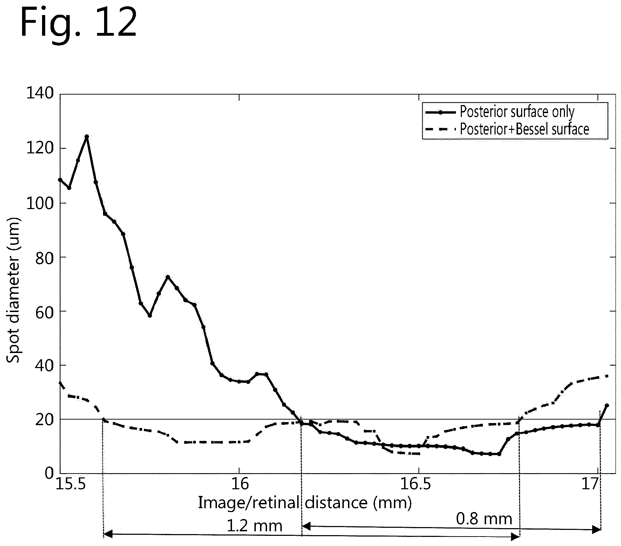

To further illustrate the effect of the presence of the diffraction generating profile, Figure 12 presents a direct comparison of the spot sizes for the first model and the second model. The first (posterior) surface **110** in both models is defined by a radius of curvature **R = -3.84mm,** a conic constant **k = -10.5,** and higher order aspherical coefficients $a_4$ = **0.00625, $a_6$ = 0.003125, $a_8$ = -0.00018867,** and $a_{10}$ = **-9.8507·10$^{-6}$.** The Bessel function used for the surface profile of the second (anterior) surface 120 in model 2 was scaled as described above with $A$ = **426nm** and $P$ = **405$\mu$m.**

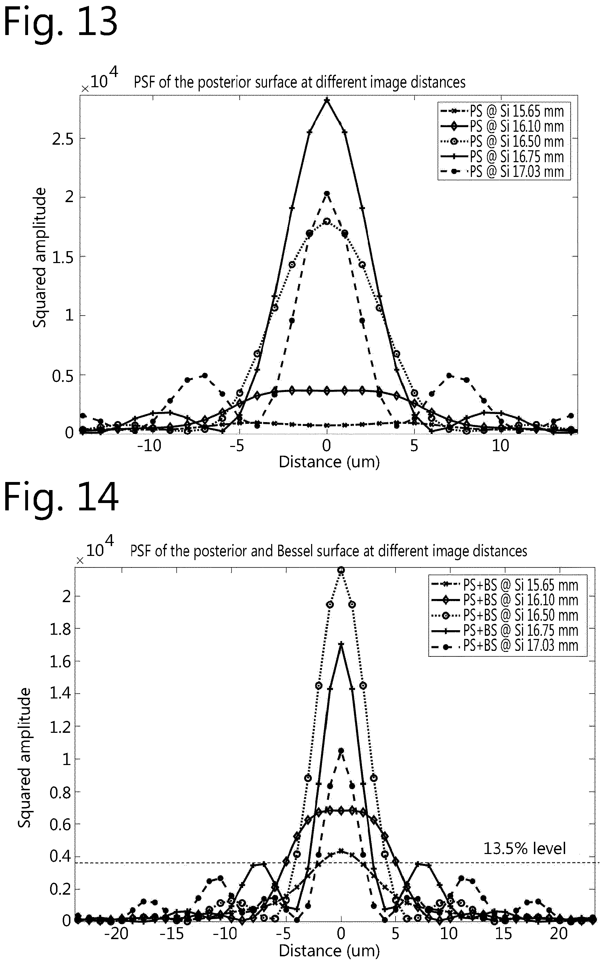

Figures 13 and 14 plot the point spread functions at different image distances for the first and the second model, respectively, whereby the model parameters are the same as those listed above in the context of Figure 12. Figure 13 (model 1, without the diffraction generating profile on the anterior surface) shows a large variation in point spread function along the propagation distance, limiting the depth of focus. Figure 14 (model 2, with the diffraction generating profile on the anterior surface) shows a much smoother variation in point spread function along the propagation distance, enlarging the depth of focus.

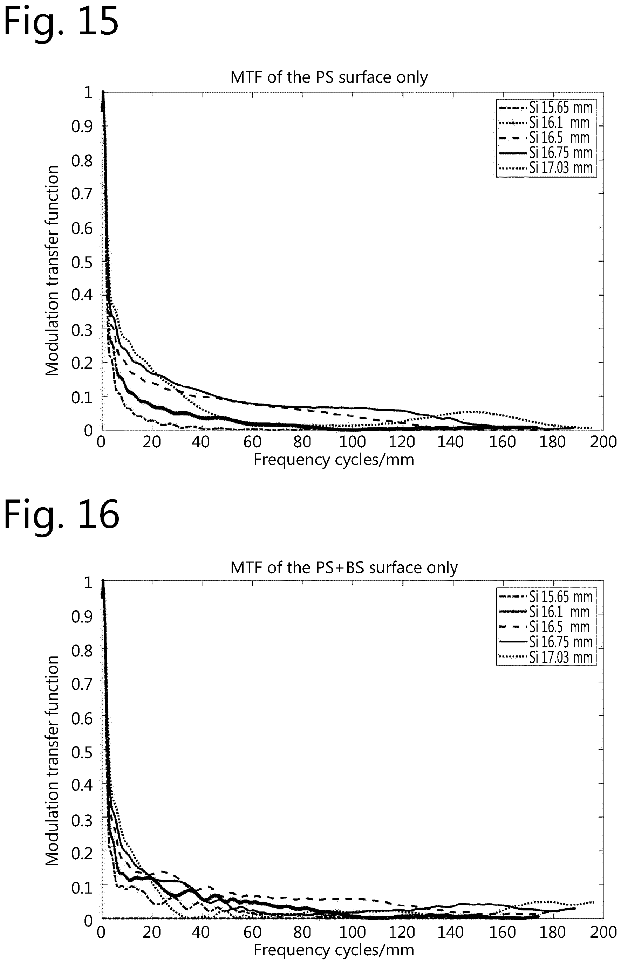

Figures 15 and 16 plot the modulation transfer functions at different image distances for the first and the second model, respectively, whereby the model parameters are the same as those listed above in the context of Figure 12. The modulation transfer function of Figure 15 (model 1, without the diffraction generating profile on the anterior surface) is larger than that of Figure 16 (model 2, with the diffraction generating profile on the anterior surface) in a narrower depth-of-focus region, but at the periphery model 2 has larger modulation transfer function values. This again shows that the presence of the diffraction generating profile on the anterior surface significantly improves the depth of focus of the intraocular lens.

The simulation results presented above show that by using the second (anterior) surface as a spatial separator instead of a refractive element, an intraocular lens according to the present invention can achieve the same overall optical power as a conventional biconvex intraocular lens (for the purpose of providing adequate distance vision) while providing increased depth of focus (for the purpose of providing improved nearby vision).

Another aspect of the present invention is the production of an intraocular lens as described above, wherein the thickness variations that define the surface profile are generated by molding or by lathing.

Another aspect of the present invention is the treatment of presbyopia or a related disorder in a patient by implanting an intraocular lens as described above in one or both eyes of the patient.

While the invention has been described hereinabove with reference to specific embodiments, this was done to illustrate and not to limit the invention, the scope of which is determined by the attached claims.

**Claims**

1. An intraocular lens (100) for contributing to the focusing of incident light onto a retina (70) of a user, the intraocular lens (100) comprising:

    - a first surface (110), and
    - a second surface (120),
    wherein said first surface (110) is configured to refractively focus said incident light, said first surface (110) exhibiting a non-uniform refractive power;
    **characterized in that** said second surface (120) is provided with a diffraction generating profile configured to spatially separate said incident light into at least a first portion having a first spatial frequency characteristic and a second portion having a second spatial frequency characteristic;
    **and in that** said spatial separation and said non-uniform refractive power of said first surface (110) cause said first portion to be refracted by said first surface (110) at a first level of refractive power and said second portion to be refracted by said first surface (110) at a second level of refractive power, said first level of refractive power and said second level of refractive power being different.

2. The intraocular lens (100) according to claim 1, shaped as a biconvex lens.

3. The intraocular lens (100) according to claim 1, shaped as a planoconvex lens, wherein said first surface (110) is convex.

4. The intraocular lens according to any of the preceding claims, wherein said non-uniform refractive power of said first surface (110) presents a characteristic of positive spherical aberration.

5. The intraocular lens according to any of the preceding claims, wherein said diffraction generating profile presents a circular symmetry.

6. The intraocular lens according to claim 5, wherein said diffraction generating profile is shaped according to a Bessel function of the first kind.

7. The intraocular lens according to claim 6, wherein said diffraction generating profile is shaped according to a zero-order Bessel function of the first kind, $z(r) = A \cdot J_0(r/P)$, wherein a radial scaling factor $P$ is in the range between 90 $\mu$m and 900 $\mu$m and a profile depth scaling factor $A$ is in the range between 100 nm and 1000 nm.

8. The intraocular lens according to any of the preceding claims, wherein the intraocular lens (100) comprises a haptic part (15, 20) that surrounds the optical part (14) of the lens (100) and further contains a groove (16) of such shape to accommodate the anterior (10) and posterior (12) capsules of the lens bag.

Figure 1

Figure 2

Figure 3

Figure 4

Figure 5

FIELD
POSITION                                              11-Oct-2021

0.00, 1.00                             ●              RMS   =      0.003289
0.000,1.500 MM                                        100%  =      0.005016

0.00, 0.00                             ●              RMS   =      0.002671
0.000,0.000 MM                                        100%  =      0.004227

0.00,-1.00                             ●              RMS   =      0.003557
0.000,-1.50 MM                                        100%  =      0.005557

                                                          .500E-01 MM

DEFOCUSING                    0.00000
     LC ON-20degree
     POSITION 3

## Figure 6

Figure 7

Figure 8

FIELD
POSITION                                    11-Oct-2021

0.00, 1.00                   RMS    =    0.008680
0.000,1.500 MM               100%  =    0.012056

0.00, 0.00                   RMS    =    0.007773
0.000,0.000 MM               100%  =    0.020723

0.00,-1.00                   RMS    =    0.018679
0.000,-1.50 MM               100%  =    0.042772

                                    .294E-01 MM

DEFOCUSING              0.00000
    LC_ON-00degree

## Figure 9

# Fig. 10

Spot size vs image distance posterior surface only

# Fig. 11

Spot size vs image distance for posterior surface with aspheric coefficient (A=750 nm, P=600 um)

# Fig. 12

# Fig. 13

PSF of the posterior surface at different image distances

Legend:
- PS @ Si 15.65 mm
- PS @ Si 16.10 mm
- PS @ Si 16.50 mm
- PS @ Si 16.75 mm
- PS @ Si 17.03 mm

# Fig. 14

PSF of the posterior and Bessel surface at different image distances

Legend:
- PS+BS @ Si 15.65 mm
- PS+BS @ Si 16.10 mm
- PS+BS @ Si 16.50 mm
- PS+BS @ Si 16.75 mm
- PS+BS @ Si 17.03 mm

13.5% level

# Fig. 15

MTF of the PS surface only

# Fig. 16

MTF of the PS+BS surface only

Europäisches
Patentamt

European
Patent Office

Office européen
des brevets

# EUROPEAN SEARCH REPORT

Application Number

EP 21 20 4739

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/115932 A2 (APTER ROBERT [CH]; APTER ALAIN [CH]) 24 September 2009 (2009-09-24) * the whole document * ----- | 1-7 | INV. G02B3/10 G02C7/06 |

**TECHNICAL FIELDS SEARCHED (IPC)**

G02B
G02C

The present search report has been drawn up for all claims

| Place of search | Date of completion of the search | Examiner |
|---|---|---|
| Munich | 1 April 2022 | Frisch, Anna Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-7

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
1. claims: 1-7

      Subject 1 relates to an intraocular lens and to optical
      details thereof
                     ---


2. claim: 8

      Subject 2 also relates to an intraocular lens and details of
      its positioning in the eye
                     ---
```

## EP 4 174 535 A1

## ANNEX TO THE EUROPEAN SEARCH REPORT
## ON EUROPEAN PATENT APPLICATION NO.

EP 21 20 4739

01-04-2022

| Patent document cited in search report | | Publication date | Patent family member(s) | | Publication date |
|---|---|---|---|---|---|
| WO 2009115932 | A2 | 24-09-2009 | EP | 2243053 A2 | 27-10-2010 |
| | | | US | 2010321635 A1 | 23-12-2010 |
| | | | WO | 2009115932 A2 | 24-09-2009 |

**EP 4 174 535 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0916320 A1